# EUROPEAN PATENT APPLICATION

(11) **EP 4 516 207 A1**
(43) Date of publication of application: **05.03.2025**
(21) Application number: 24197102.7
(22) Date of filing: 28.08.2024
(51) Int. Cl.: A61B 3/16

(54) **CONTACTLESS OPHTHALMOTONOMETER AND CONTACTLESS OPHTHALMOTONOMETER ACTUATING METHOD**

(30) Priority: 01.09.2023 JP 2023142194
(71) Applicant: Topcon Corporation, Tokyo 174-8580 (JP)
(72) Inventor: KOSHIHARA, Shiori, Tokyo, 1748580 (JP); NAKAMURA, Satoshi, Tokyo, 1748580 (JP); FURUGAICHI, Taketo, Tokyo, 1748580 (JP); HARADA, Akihiro, Tokyo, 1748580 (JP)
(74) Representative: Schmitt-Nilson Schraud Waibel Wohlfrom Patentanwälte Partnerschaft mbB

(57) **Abstract**

A contactless ophthalmotonometer includes a cylinder, a piston movably provided inside the cylinder, an actuator (solenoid) configured to move the piston inside the cylinder by applying current to compress air inside the cylinder, a nozzle configured to spray the air compressed with the piston to a subject eye, and a current application control unit (measurement control unit) configured to apply the current to the actuator, wherein the current application control unit performs first application processing for applying the current of a first current value to the actuator and, when a predetermined switching condition is satisfied while the first application processing is being performed, the first application processing is switched to second application processing for applying the current of a second current value higher than the first current value to the actuator.

## Description

### BACKGROUND OF THE INVENTION

### Field of the Invention

Aspects of the technology described herein relate to a contactless ophthalmotonometer to contactlessly measure an intraocular pressure value of a subject eye and a contactless ophthalmotonometer actuating method.

### Description of the Related Art

A contactless ophthalmotonometer has been known (see Japanese Patent Application Laid-Open No. 2022-111333, for example) which contactlessly measures an intraocular pressure of a subject eye. The contactless ophthalmotonometer is provided with a spraying mechanism which sprays air to a cornea of the subject eye via a nozzle. In this way, the contactless ophthalmotonometer drives the spraying mechanism to spray air from the nozzle to the cornea of the subject eye, causing deformation of the cornea of the subject eye. Then, the contactless ophthalmotonometer detects the deformed state to measure an intraocular pressure value of the subject eye without contacting the cornea.

The spraying mechanism of the contactless ophthalmotonometer according to PTL1 includes a cylinder, a piston, a nozzle, a chamber, and a rotary solenoid. The piston is provided movably within the cylinder. The nozzle is connected to the chamber and is communicated to the inside (pressurizing chamber) of the cylinder through an element such as the chamber. The rotary solenoid is connected to the piston through a crank mechanism that converts a rotary motion of the rotary solenoid to a linear motion. The rotary solenoid rotates by current application so that the piston is moved within the cylinder through the crank mechanism. In this way, the air inside the cylinder compressed by the piston jets out from the nozzle toward the cornea of the subject eye through the element such as the chamber.

### Citation List

PTL 1: Japanese Patent Application Laid-Open No. 2022-111333

### SUMMARY OF THE INVENTION

The contactless ophthalmotonometer described in PTL1 drives the rotary solenoid with current to spray the air from the nozzle to the cornea of the subject eye. When large noise is generated by the spraying mechanism due to the driven rotary solenoid, the large noise may make the subject uncomfortable. Thus, there has been a demand for reducing the noise generated by the spraying mechanism. In order to reduce the noise generated by the spraying mechanism, it is required to reduce the current value of the current applied to the rotary solenoid.

However, the reduction of the current value of the current applied to the rotary solenoid may cause gradual reduction of the force to the piston through the crank mechanism as the rotation of the rotary solenoid advances, although the piston moves as intended upon initial operation of the rotary solenoid. Therefore, the pressure of the air within the chamber increases insufficiently, and the pressure of the air to be sprayed to the cornea of the subject eye from the nozzle may be insufficient. In this case, an intraocular pressure value of the subject eye cannot be measured with high precision.

The aspects of the technology described herein have been made in view of these circumstances. And, the object of the aspects of the technology described herein is to provide a contactless ophthalmotonometer and a contactless ophthalmotonometer actuating method that enable both of noise reduction during measurement of an intraocular pressure and precision of the measurement.

Some embodiments provide for a contactless ophthalmotonometer for achieving the object of the aspects of the technology described herein. The contactless ophthalmotonometer includes a cylinder, a piston movably provided inside the cylinder, an actuator configured to move the piston inside the cylinder with current applied to the actuator to compress air inside the cylinder with the piston, a nozzle in communication with the inside of the cylinder, the nozzle configured to spray the air compressed with the piston to a subject eye, and a current application control unit configured to apply the current to the actuator, wherein the current application control unit performs first application processing for applying the current of a first current value to the actuator and, when a predetermined switching condition is satisfied while the first application processing is being performed, the first application processing is switched to second application processing for applying the current of a second current value higher than the first current value to the actuator.

The contactless ophthalmotonometer seeks to reduce the noise by applying current of a first current value to the actuator in the first application processing and increases the current value to be applied to the actuator to the second current value when a predetermined switching condition is satisfied so that a decrease in precision of the measurement of an intraocular pressure value of the subject eye can be prevented.

In the contactless ophthalmotonometer according to another aspect, the nozzle is in communication with the inside of the cylinder through a chamber in communication with the inside of the cylinder, the contactless ophthalmotonometer further includes a pressure sensor configured to detect pressure inside the chamber, and the current application control unit determines whether the switching condition is satisfied or not based on whether the pressure detected by the pressure sensor achieves a predetermined pressure threshold. Thus, a decrease in pressure of the air sprayed to the subject eye E is prevented.

The contactless ophthalmotonometer according to another aspect further includes a detection sensor configured to detect a displaced amount of the actuator, wherein the current application control unit determines whether the switching condition is satisfied or not based on whether the displaced amount detected by the detection sensor achieves a predetermined displaced amount threshold. Thus, a decrease in pressure of the air sprayed to the subject eye is prevented.

In the contactless ophthalmotonometer according to another aspect, the current application control unit increases the second current value in a stepwise manner while the second application processing is being performed.

In the contactless ophthalmotonometer according to another aspect, the nozzle is in communication with the inside of the cylinder through a chamber in communication with the inside of the cylinder, the contactless ophthalmotonometer further includes a pressure sensor that detects pressure inside the chamber, and the current application control unit increases the second current value in a stepwise manner in accordance with an increase in the pressure detected by the pressure sensor while the second application processing is being performed.

The contactless ophthalmotonometer according to another aspect further includes a detection sensor configured to detect a displaced amount of the actuator, and the current application control unit increases the second current value in a stepwise manner in accordance with an increase in the displaced amount detected by the detection sensor while the second application processing is being performed.

In the contactless ophthalmotonometer according to another aspect, the current application control unit stops the second application processing when a defined period of time determined in advance passes from start of the first application processing.

A contactless ophthalmotonometer according to another aspect further includes a projecting optical system configured to project a luminous flux to the subject eye while the air is being sprayed from the nozzle to the subject eye, and a receiving optical system configured to receive reflected light of the luminous flux reflected at the subject eye and outputs an applanation signal while the air is being sprayed from the nozzle to the subject eye, and the current application control unit stops the second application processing when a peak of the applanation signal output from the receiving optical system is detected while the second application processing is being performed. Thus, so-called soft puff that reduces the amount of air to be sprayed to the subject eye can be achieved so that unpleasantness of the subject can be reduced.

In a contactless ophthalmotonometer according to another aspect, the actuator is a rotary actuator configured to cause the piston to move inside the cylinder through a crank mechanism.

In a method for actuating a contactless ophthalmotonometer for achieving the object of the aspects of the technology described herein, the contactless ophthalmotonometer including a cylinder, a piston movably provided inside the cylinder, an actuator configured to move the piston inside the cylinder with current applied to the actuator to compress air inside the cylinder with the piston, a nozzle in communication with the inside of the cylinder, the nozzle configured to spray the air compressed with the piston to a subject eye. The method includes: performing first application processing for applying the current of a first current value to the actuator when the current is to be applied to the actuator; and switching the first application processing to second application processing for applying the current of a second current value higher than the first current value to the actuator when a predetermined switching condition is satisfied while the first application processing is being performed.

The aspects of the technology described herein enable both of noise reduction during measurement of an intraocular pressure and precision of the measurement.

### BRIEF DESCRIPTION OF THE DRAWINGS

Various aspects and embodiments of the application will be described with reference to the following figures. Items appearing in multiple figures are indicated by the same or a similar reference number in all the figures in which they appear.
Fig. 1 is a lateral view of a contactless ophthalmotonometer according to a first embodiment;
Fig. 2 is a top schematic diagram of some types of optical systems within a measurement head illustrated from top view;
Fig. 3 is a lateral schematic diagram of some types of optical systems within the measurement head illustrated from lateral view;
Fig. 4 is a functional block diagram of a control device;
Fig. 5 is a diagram illustrating an example of a current waveform that is a waveform of current to be applied to a solenoid by a measurement control unit during spraying processing;
Fig. 6 is a flowchart illustrating a flow of intraocular pressure measurement processing on a subject eye that is a method for actuating a contactless ophthalmotonometer according to the first embodiment;
Fig. 7 is a graph illustrating an applanation waveform based on an applanation signal output continuously from an applanation detecting optical system;
Fig. 8 is an explanatory diagram for explaining a stop timing of second application processing by the contactless ophthalmotonometer according to a second embodiment;
Fig. 9 is a flowchart illustrating a flow of intraocular pressure measurement processing on a subject eye by a contactless ophthalmotonometer according to the second embodiment; and
Fig. 10 is an explanatory diagram for explaining the second application processing to be performed by a contactless ophthalmotonometer according to a third embodiment.

### DETAILED DESCRIPTION OF THE EMBODIMENTS

### First Embodiment

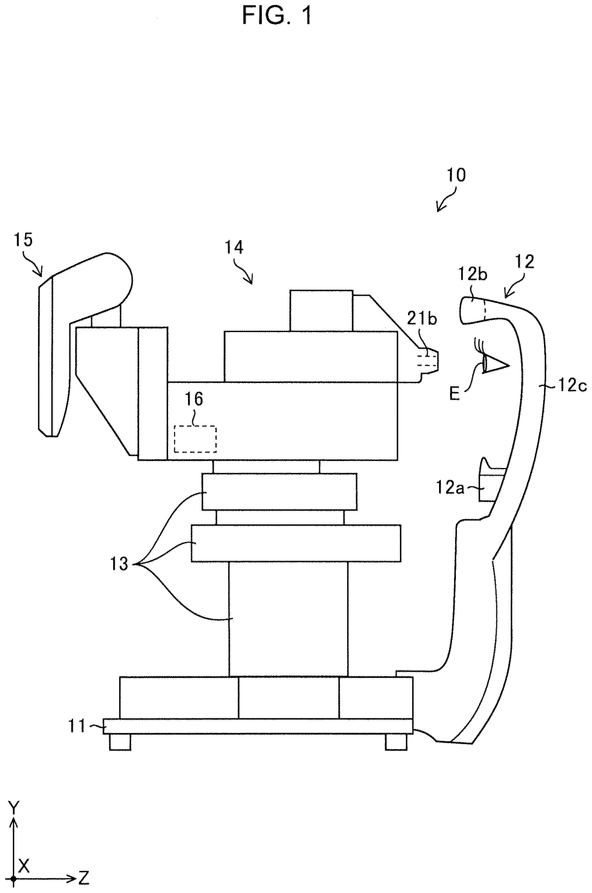

Fig. 1 is a lateral view of a contactless ophthalmotonometer 10 according to a first embodiment. As illustrated in Fig. 1, the contactless ophthalmotonometer 10 contactlessly measures an intraocular pressure value of a subject eye E. This contactless ophthalmotonometer 10 includes a base 11, a face supporting unit 12, a moving mechanism 13, a measurement head 14, a monitor 15, and a control device 16.

XYZ directions (three axial directions) in the figure are orthogonal to each other. The Y direction is a top-bottom direction. The Z direction is a front-back direction (which is also called "working distance direction") closer to or away from a subject (subject eye E). The X direction is the right-left direction perpendicular to both of the top-bottom direction and the front-back direction. Also, the side closer to the subject eye E (subject) in the Z direction (front-back direction) is called a Z direction front side, and the side away from the subject eye E (subject) is called a Z direction back side.

On the base 11, the face supporting unit 12 and the moving mechanism 13 are provided from the Z direction front side to the Z direction back side. The base 11 supports the measurement head 14 movably in the XYZ directions through the moving mechanism 13.

The face supporting unit 12 is fixed to the base 11. This face supporting unit 12 includes a jaw receiver 12a which receives the jaw of a subject, a forehead protector 12b against which the forehead of the subject is abutted, and a pair of support rods 12c which supports the forehead protector 12b. And, the face supporting unit 12 supports the face of the subject.

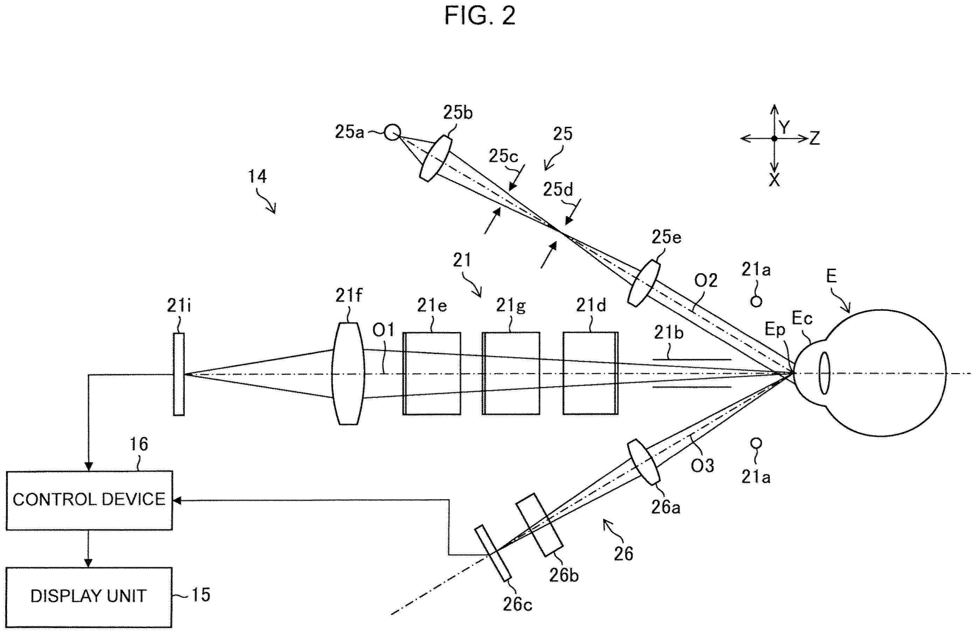

The measurement head 14 sprays air from the nozzle 21b to the cornea Ec (see Fig. 2) of the subject eye E to deform the cornea Ec. And, the measurement head 14 detects the deformed state to contactlessly measure an intraocular pressure value of the subject eye E.

The monitor 15 is, for example, a touch-panel type monitor. The monitor 15 is attached to the back side of the measurement head 14, for example. This monitor 15 displays various kinds of images such as a captured image (observation image) of the subject eye E captured by the measurement head 14, a measurement result with respect to the intraocular pressure value of the subject eye E, and/or a menu screen to be used for performing various kinds of operation and various kinds of setting, under control of the control device 16, which is described later.

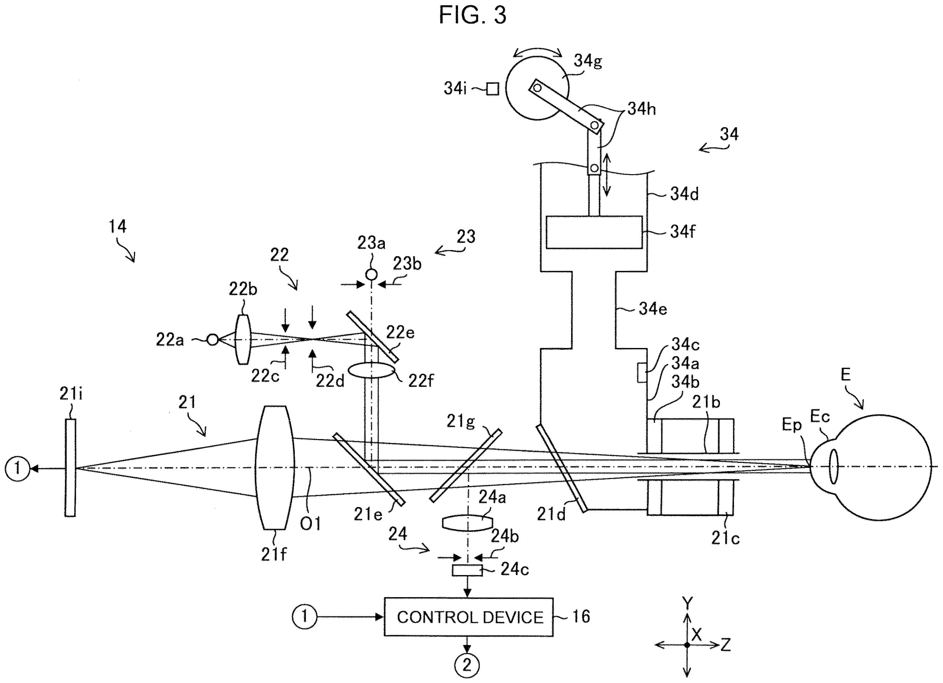

Fig. 2 is a top schematic diagram of some types of optical systems within the measurement head 14 illustrated from top view (Y direction). Fig. 3 is a lateral schematic diagram of some types of optical systems within the measurement head 14 illustrated from lateral view (X direction).

As illustrated in Figs. 2 and 3, the measurement head 14 includes anterior eye part observing optical system 21, an XY alignment indicator projecting optical system 22, a fixation target projecting optical system 23, an applanation detecting optical system 24, a Z alignment indicator projecting optical system 25, a Z alignment detecting optical system 26, and a spraying mechanism 34.

The anterior eye part observing optical system 21 is used for observation of the anterior eye part of the subject eye E and for XY alignment of the measurement head 14 in the XY directions with respect to the subject eye E. This anterior eye part observing optical system 21 includes anterior eye part illumination light sources 21a (see Fig. 2). Also, on the optical axis O1 of the anterior eye part observing optical system 21, a nozzle 21b for spraying air, an anterior eye part window glass 21c (see Fig. 3) which holds a tip of the nozzle 21b, a chamber window glass 21d, a half-mirror 21e, a half-mirror 21g, an objective lens 21f, and an image-capturing element 21i are provided.

The anterior eye part illumination light sources 21a are provided at positions around the anterior eye part window glass 21c and directly illuminate an anterior eye part of the subject eye E.

The nozzle 21b connects to a chamber 34a (see Fig. 3) in the spraying mechanism 34 and sprays air to the cornea Ec of the subject eye E.

An image of the anterior eye part (image light from the anterior eye part) of the subject eye E passes outside the nozzle 21b, passes through the anterior eye part window glass 21c, a glass plate 34b, which is described later, the chamber window glass 21d, the half-mirror 21g, and the half-mirror 21e and is formed on a light receiving surface of the image-capturing element 21i via the objective lens 21f.

As the image-capturing element 21i, a charge coupled device (CCD) type or a complementary metal oxide semiconductor (CMOS) type image sensor, for example, may be used. This image-capturing element 21i captures an image of the anterior eye part incident on the light receiving surface thereof, produces a captured-image signal, and outputs the captured-image signal to the control device 16. Thus, the control device 16 displays an observation image of the anterior eye part of the subject eye E based on the captured-image signal output from the image-capturing element 21i, on the monitor 15.

Also, the anterior eye part observing optical system 21 guides reflected light from the cornea Ec of the XY-alignment indicating light projected from the XY alignment indicator projecting optical system 22, which is described later, to the subject eye E toward the light receiving surface of the image-capturing element 21i. The reflected light passes through the nozzle 21b, the chamber window glass 21d, the half-mirror 21g, and the half-mirror 21e and is formed into an image on the light receiving surface of the image-capturing element 21i via the objective lens 21f. Thus, on the light receiving surface of the image-capturing element 21i, an XY spot is formed at a position according to the positional relationship (relative position) in the XY directions between the measurement head 14 and the cornea Ec. As a result, XY alignment detection can be implemented which detects the relative position in the XY directions of subject eye E with respect to the measurement head 14.

The image-capturing element 21i captures an image of the XY spot formed on the light receiving surface thereof and outputs the captured-image signal of the XY spot to the control device 16. Thus, the control device 16 superimposes the image of the XY spot on the observation image of the anterior eye part displayed on the monitor 15. An alignment aiding mark may also be displayed on the monitor 15.

The XY alignment indicator projecting optical system 22 corresponds to the projecting optical system of the aspects of the technology described herein. The XY alignment indicator projecting optical system 22 projects XY-alignment indicating light corresponding to the luminous flux of the aspects of the technology described herein to the cornea Ec of the subject eye E from the front. The XY-alignment indicating light is used for XY alignment of the measurement head 14 relative to the subject eye E. Also, the XY-alignment indicating light is also used for measurement of an intraocular pressure value of the subject eye E. Hereinafter, the reflected light of the XY-alignment indicating light by the cornea Ec is simply called "XY indicator reflected light" for short.

The XY alignment indicator projecting optical system 22 includes a XY alignment light source 22a, a condensing lens 22b, an aperture diaphragm 22c, a pinhole plate 22d, a dichroic mirror 22e, and a collimator lens 22f (see Fig. 3). Here, the XY alignment indicator projecting optical system 22 shares the half-mirror 21e with the anterior eye part observing optical system 21.

The XY alignment light source 22a emits infrared light. The collimator lens 22f is placed on the optical path of the XY alignment indicator projecting optical system 22 such that the focus of the collimator lens 22f is matched with the pinhole plate 22d. The infrared light emitted from the XY alignment light source 22a passes through the aperture diaphragm 22c while being focused by the condensing lens 22b and is guided to a hole of the pinhole plate 22d.

The infrared light having passed through the hole of the pinhole plate 22d is reflected by the dichroic mirror 22e, and is guided toward the collimator lens 22f. Then, after the infrared light is changed to parallel light via the collimator lens 22f, the parallel light of the infrared light is emitted from the collimator lens 22f to the half-mirror 21e. After the parallel light of the infrared light is reflected by the half-mirror 21e, the parallel light advances along the optical axis O1 of the anterior eye part observing optical system 21. Thus, after the parallel light of the infrared light is transmitted through the half-mirror 21g and the chamber window glass 21d, the parallel light passes through inside of the nozzle 21b so that the parallel light enters the subject eye E as XY-alignment indicating light.

The XY-alignment indicating light entering the subject eye E is reflected by the surface of the cornea Ec and forms an XY spot, not illustrated. Here, the aperture diaphragm 22c is provided at a position conjugated with a cornea vertex Ep of the cornea Ec with respect to the collimator lens 22f.

The fixation target projecting optical system 23 projects a fixation target to the subject eye E. This fixation target projecting optical system 23 has a fixation target light source 23a and a pinhole plate 23b (see Fig. 3). Also, the fixation target projecting optical system 23 shares the dichroic mirror 22e and collimator lens 22f with the XY alignment indicator projecting optical system 22 and shares the half-mirror 21e with the anterior eye part observing optical system 21.

The fixation target light source 23a emits visible light as fixation target light. This fixation target light is guided toward the hole of the pinhole plate 23b, is transmitted through the hole of the pinhole plate 23b and the dichroic mirror 22e and is then emitted to the collimator lens 22f. Then, the fixation target light is changed to substantially parallel light via the collimator lens 22f, is emitted to the half-mirror 21e, and is reflected by the half-mirror 21e so that the fixation target light advances along the optical axis O1 of the anterior eye part observing optical system 21. Thus, after the fixation target light is transmitted through the half-mirror 21g and the chamber window glass 21d, the fixation target light passes through inside of the nozzle 21b and reaches the subject eye E. By allowing a subject to concentrate on the fixation target as a mark for visual fixation, the line of sight of the subject can be fixed.

The applanation detecting optical system 24 (see Fig. 3) corresponds to the light receiving optical system of the aspects of the technology described herein and receives XY indicator reflected light and outputs an applanation signal (also called "cornea deformation signal") that is a received light signal indicating the quantity of the XY indicator reflected light. The applanation detecting optical system 24 has a lens 24a, a pinhole plate 24b, and a received light sensor 24c and shares the half-mirror 21g with the anterior eye part observing optical system 21.

The lens 24a focuses the XY indicator reflected light to an aperture of the pinhole plate 24b when the surface of the cornea Ec is a flat surface. The aperture of the pinhole plate 24b is provided at a focal position of the lens 24a.

The received light sensor 24c is, for example, a photodiode which outputs an applanation signal according to the received quantity of the XY indicator reflected light. This received light sensor 24c outputs the applanation signal to the control device 16.

The XY indicator reflected light passes through inside of the nozzle 21b, is transmitted through the chamber window glass 21d, and reaches the half-mirror 21g. Then, a part of the XY indicator reflected light is reflected by the half-mirror 21g and then enters the pinhole plate 24b through the lens 24a.

When the cornea Ec is changed to have a flat applanation state (flat state) in which the surface of the cornea is flattened by the air spray from the nozzle 21b, the applanation detecting optical system 24 causes the entire XY indicator reflected light coming to the applanation detecting optical system 24 to reach the received light sensor 24c through the pinhole plate 24b. Also, the applanation detecting optical system 24, when the cornea Ec has a state other than an applanation state, partially blocks off the XY indicator reflected light with the pinhole plate 24b and causes the light to reach the received light sensor 24c. Therefore, the signal intensity of the detection signal of the XY indicator reflected light output from the applanation detecting optical system 24 gradually increases as the surface of the cornea Ec changes from the convex state to an applanation state and gradually decreases as it further changes from the applanation state to a concave state.

The Z alignment indicator projecting optical system 25 (see Fig. 2) projects Z alignment indicating light for Z alignment in the Z axis direction from a diagonal direction with respect to the cornea Ec. The Z alignment indicator projecting optical system 25 includes, along on an optical axis 02, a Z alignment light source 25a, a condensing lens 25b, an aperture diaphragm 25c, a pinhole plate 25d, and a collimator lens 25e.

The Z alignment light source 25a emits infrared light (having a wavelength of 860 nm, for example). The aperture diaphragm 25c is provided at a position conjugated with a cornea vertex Ep with respect to the collimator lens 25e. The collimator lens 25e is placed such that its focal point is matched with the hole of the pinhole plate 25d.

The infrared light emitted from the Z alignment light source 25a passes through the aperture diaphragm 25c while being focused by the condensing lens 25b and advances to the pinhole plate 25d. Then, the infrared light having passed through the hole of the pinhole plate 25d is changed to parallel light at the collimator lens 25e and then enters the subject eye E as Z alignment indicating light and is reflected by the cornea Ec so that a bright spot image is formed on the subject eye E.

The Z alignment detecting optical system 26 receives the reflected light from the cornea Ec of the Z alignment indicating light (hereinafter, called "Z indicator reflected light" for short) and detects a positional relationship in the Z axis direction between the measurement head 14 and the cornea Ec. This Z alignment detecting optical system 26 includes, along on the optical axis 03, an image-forming lens 26a, a cylindrical lens 26b, and a received light sensor 26c.

As the cylindrical lens 26b, one having power in the Y axis direction is used. The received light sensor 26c is a sensor capable of detecting a light received position of the Z indicator reflected light at a light receiving surface of the received light sensor 26c and is, for example, a line sensor or a position sensitive detector (PSD).

The Z indicator reflected light is focused at the image-forming lens 26a and then advances to the cylindrical lens 26b. Then, the Z indicator reflected light is focused in the Y axis direction via the cylindrical lens 26b so that a bright spot image is formed on the received light sensor 26c.

The received light sensor 26c has a conjugated positional relationship with the aforementioned bright spot image formed on the subject eye E by the Z alignment indicator projecting optical system 25 with respect to the image-forming lens 26a within the XZ plane. Also, the received light sensor 26c has a conjugated positional relationship with the cornea vertex Ep with regard to the image-forming lens 26a and the cylindrical lens 26b within the YZ plane. In other words, since the received light sensor 26c has a conjugated relationship with the aperture diaphragm 25c, the Z indicator reflected light on the surface of the cornea Ec efficiently enters the received light sensor 26c even when the cornea Ec is displaced in the Y direction. Then, the received light sensor 26c outputs a detection signal for the Z indicator reflected light condensed by the cylindrical lens 26b (hereinafter, called "Z detection signal") to the control device 16. This Z detection signal indicates a relative position in the Z direction of the subject eye E with respect to the measurement head 14, more specifically, a distance in the Z direction between the measurement head 14 and the subject eye E (cornea vertex Ep). Thus, the Z alignment detection can be implemented.

The spraying mechanism 34 (see Fig. 3) includes, in addition to the nozzle 21b described above, a chamber 34a, a glass plate 34b, a pressure sensor 34c, a cylinder 34d, a communicating tube 34e, a piston 34f, a solenoid 34g, a crank mechanism 34h, and an angle detection sensor 34i.

The nozzle 21b is attached to the chamber 34a through a transparent glass plate 34b. Also, inside the chamber 34a, the chamber window glass 21d is provided at a position facing the nozzle 21b. Further, inside the chamber 34a, the pressure sensor 34c is provided. This pressure sensor 34c outputs a pressure detection signal which indicates pressure (internal pressure) inside the chamber 34a to the control device 16.

The cylinder 34d connects to a chamber 34a through the communicating tube 34e. Thus, since the inside of cylinder 34d and the inside of the chamber 34a are communicated through the communicating tube 34e, the inside of the cylinder 34d and the nozzle 21b are communicated. Also, the piston 34f is movably provided inside the cylinder 34d. These cylinder 34d and piston 34f configure a compression space for air.

The solenoid 34g is a rotary solenoid which is rotated by application of current and corresponds to the actuator and the rotary actuator of the aspects of the technology described herein. This solenoid 34g is connected to the piston 34f through the crank mechanism 34h that converts a rotary motion of the solenoid 34g to a linear motion. This solenoid 34g rotates when current is applied thereto so that the piston 34f is moved linearly within the cylinder 34d through the crank mechanism 34h under control of the control device 16 to compress the air inside the cylinder 34d. Thus, through the communicating tube 34e and the chamber 34a, air is sprayed from the nozzle 21b to the cornea Ec.

In the spraying mechanism 34, the internal pressure of the chamber 34a is detected by the pressure sensor 34c, and thereby the pressure of the air when air is sprayed from the nozzle 21b to the cornea Ec can be acquired.

The angle detection sensor 34i corresponds to the detection sensor of the aspects of the technology described herein and detects a rotation angle of the solenoid 34g corresponding to the displaced amount of the actuator of the aspects of the technology described herein and outputs the detection result to the control device 16. The rotation angle of the solenoid 34g detected by the angle detection sensor 34i indirectly indicates the internal pressure of the chamber 34a and can be utilized for driving control over the solenoid 34g the details of which are described later. When the detection result of the angle detection sensor 34i is not utilized for the driving control over the solenoid 34g, the angle detection sensor 34i can be omitted.

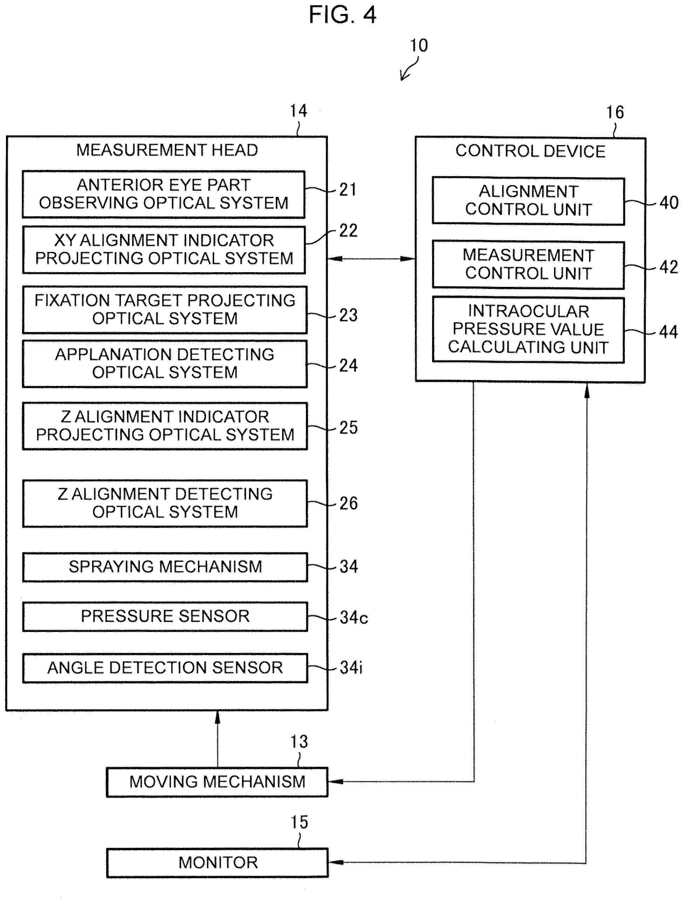

Fig. 4 is a functional block diagram of the control device 16. As illustrated in Fig. 4, the control device 16 generally controls operations by the contactless ophthalmotonometer 10. The control device 16 includes an arithmetic circuit including various kinds of processors and a memory. The various kinds of processors may include processors such as a central processing unit (CPU), a graphics processing unit (GPU), an application specific integrated circuit (ASIC), a programmable logic device (such as simple programmable logic devices (SPLD), complex programmable logic device (CPLD), and field programmable gate arrays (FPGA)). Various kinds of functions of the control device 16 may be implemented by one processor or may be implemented by processors of the same kind or of different kinds.

For example, the moving mechanism 13, the measurement head 14 and the monitor 15 described above are connected to the control device 16. This control device 16 generally controls operations by the contactless ophthalmotonometer 10.

The control device 16 reads out and executes a control program, not illustrated, to function as an alignment control unit 40, a measurement control unit 42, and an intraocular pressure value calculating unit 44. Here, a term "- unit" in the description of the control device 16 may refer to "- circuit", "- device", or "- apparatus". In other words, each of those described as "- unit" may be configured by any of firmware, software, and hardware or a combination thereof.

The alignment control unit 40 operates for intraocular pressure measurement on the subject eye E and controls auto-alignment in the XYZ directions of the measurement head 14 with respect to the subject eye E.

More specifically, the alignment control unit 40 controls the anterior eye part observing optical system 21 and XY alignment indicator projecting optical system 22 described above to perform XY alignment detection which detects the relative position in the XY directions of subject eye E with respect to the measurement head 14. Also, the alignment control unit 40 controls the Z alignment indicator projecting optical system 25 and the Z alignment detecting optical system 26 to perform Z alignment detection which detects the relative position in the Z direction of the subject eye E with respect to the measurement head 14. Since the XY alignment detection and the Z alignment detection of the contactless ophthalmotonometer 10 are publicly known technologies, specific descriptions thereon are omitted here. Then, based on the XYZ alignment detection result, the alignment control unit 40 drives the moving mechanism 13 so as to perform an auto-alignment in the XYZ directions of the measurement head 14 relative to the subject eye E.

The measurement control unit 42 actuates the spraying mechanism 34 after completion of the auto-alignment, and spray processing is performed which sprays air from the nozzle 21b of the spraying mechanism 34 to the cornea Ec. More specifically, the measurement control unit 42 applies current to the solenoid 34g and rotationally drives (current drive) the solenoid 34g so that the piston 34f is moved to the farther side inside the cylinder 34d via the crank mechanism 34h. Therefore, the measurement control unit 42 corresponds to the current application control unit of the aspects of the technology described herein. At that time, the measurement control unit 42 applies current to the solenoid 34g under a condition that both of reduction of noise due to the spraying mechanism 34 and precision of the intraocular pressure measurement are enabled.

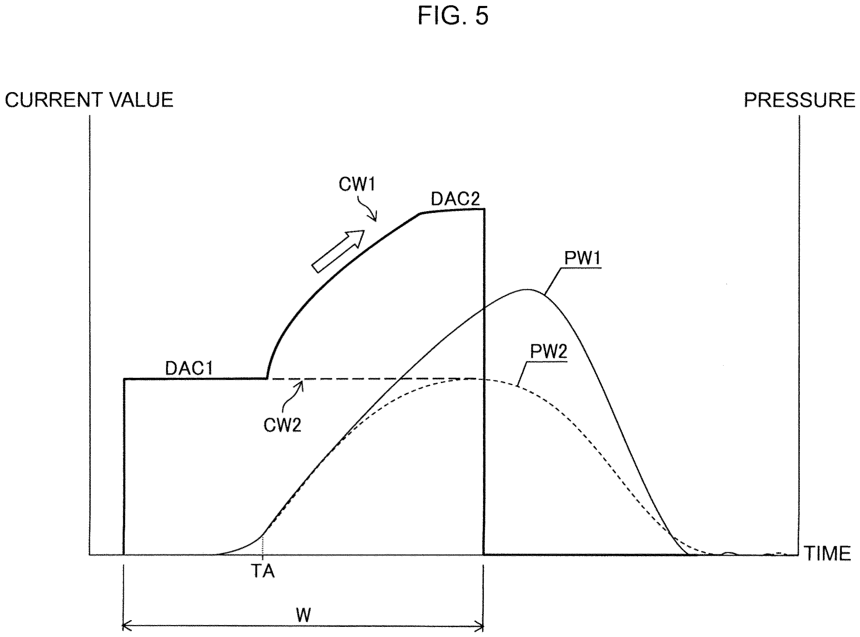

Fig. 5 is a diagram illustrating an example of a current waveform CW1 that is a waveform of current to be applied to a solenoid 34g by a measurement control unit 42 during spraying processing.

The reference character CW2 in Fig. 5 designates an example of a current waveform in a comparative example in which the reduction of noise due to the spraying mechanism 34 is enabled. Also, the reference character PW1 in Fig. 5 designates an example of an internal pressure waveform indicating a temporal change of pressure (internal pressure) inside the chamber 34a, which is detected by the pressure sensor 34c, when the measurement control unit 42 applies the current of the current waveform CW1 to the solenoid 34g. Further, the reference character PW2 in Fig. 5 designates an internal pressure waveform indicating a temporal change of pressure inside the chamber 34a, which is detected by the pressure sensor 34c, when the measurement control unit 42 applies the current of the current waveform CW2 to the solenoid 34g. Furthermore, the reference character W in Fig. 5 designates a pulse width of the current waveforms CW1, CW2, that is, the period in which current is applied to the solenoid 34g.

As illustrated in Fig. 5, in order to reduce the noise due to the spraying mechanism 34 during the spray processing, the current value of the current applied to the solenoid 34g by the measurement control unit 42 is required to be reduced. Thus, the measurement control unit 42 applies a first current value DAC1 to the solenoid 34g. The first current value DAC1 is a current value for the purpose of reduction of noise due to the spraying mechanism 34, for example, the lowest possible current value which can drive the piston 34f. This first current value DAC1 is determined properly in an experiment or a simulation, for example.

At that time, as illustrated in the current waveform CW2 in the comparative example, when the current value of the current applied to the solenoid 34g is kept constantly at the first current value DAC1 over the entire period of the pulse width W, the torque produced from the solenoid 34g is constant. On the other hand, as the rotation of the solenoid 34g advances, the force required for compressing the air in the chamber 34a increases. For that, when the current value of the current applied to the solenoid 34g is kept constant at the first current value DAC1, the degree of application of the force to the piston 34f through the crank mechanism 34h decreases as the rotation of the solenoid 34g advances. As a result, as indicated in the internal pressure waveform PW2 in the comparative example, the pressure of the air inside the chamber 34a does not increase, and the pressure of the air to be sprayed from the nozzle 21b to the cornea Ec becomes insufficient. In this case, an intraocular pressure value of the subject eye E cannot be measured with high precision.

Accordingly, the measurement control unit 42 performs first application processing for keeping the current value of the current applied to the solenoid 34g constant at the first current value DAC1 upon initial operation of the spraying processing, as illustrated in the current waveform CW1. Then, the measurement control unit 42 performs second application processing for increasing the current value of the current to be applied to the solenoid 34g to a second current value DAC2 higher than the first current value DAC1 in switching timing TA when a predetermined switching condition is satisfied while the first application processing is being performed and applies current of the second current value DAC2 to the solenoid 34g.

The switching condition for switching from the first application processing to the second application processing (hereinafter, simply called "switching condition" for short) is defined such that the force to be conducted from the solenoid 34g to the piston 34f is prevented from decreasing even when the rotation of the solenoid 34g advances. More specifically, according to this embodiment, when the pressure detected by the pressure sensor 34c while the first application processing is being performed achieves a predetermined pressure threshold, the first application processing is switched to the second application processing. Therefore, the measurement control unit 42 continues the first application processing from start of the first application processing upon initial operation of the spray processing to the time when the pressure detected by the pressure sensor 34c achieves the pressure threshold.

Then, the measurement control unit 42 performs switching from the first application processing to the second application processing by increasing the current value to be applied to the solenoid 34g from the first current value DAC1 to the second current value DAC2 when the pressure detected by the pressure sensor 34c while the first application processing is being performed achieves the pressure threshold (switching timing TA). Thus, the slope of the internal pressure waveform PW1 can be kept substantially constant, and the pressure of the air inside the chamber 34a can be increased sufficiently. The pressure threshold and the second current value DAC2 are set to values that can keep the slope of the internal pressure waveform PW1 substantially constant through an experiment or a simulation, for example.

Here, a correlation exists between a pressure detected by the pressure sensor 34c during spray processing and the rotation angle of the solenoid 34g detected by the angle detection sensor 34i. Therefore, instead of the aforementioned switching condition, switching to the second application processing may be performed when the rotation angle detected by the angle detection sensor 34i achieves a predetermined angle threshold (displaced amount threshold of the aspects of the technology described herein) while the first application processing is being performed. In this case, the measurement control unit 42 keeps the first application processing until the rotation angle detected by the angle detection sensor 34i achieves an angle threshold and switches to the second application processing in timing (switching timing TA) when the rotation angle detected by the angle detection sensor 34i achieves an angle threshold.

In a case where a defined period of time that is determined in advance has passed from start of the first application processing (period of time corresponding to a pulse width W), the measurement control unit 42 stops current application to the solenoid 34g to stop the second application processing. The measurement control unit 42 may stop the second application processing when a defined period of time determined in advance passes by starting clocking from start of the second application processing. In other words, the clocking start timing for the aforementioned defined period of time is not particularly limited.

Referring back to Fig. 4, while spray processing is being performed, the measurement control unit 42 continuously causes processing of projecting XY-alignment indicating light to the cornea Ec by the XY alignment indicator projecting optical system 22 and signal output processing including receiving XY indicator reflected light and outputting an applanation signal by the applanation detecting optical system 24 to be performed.

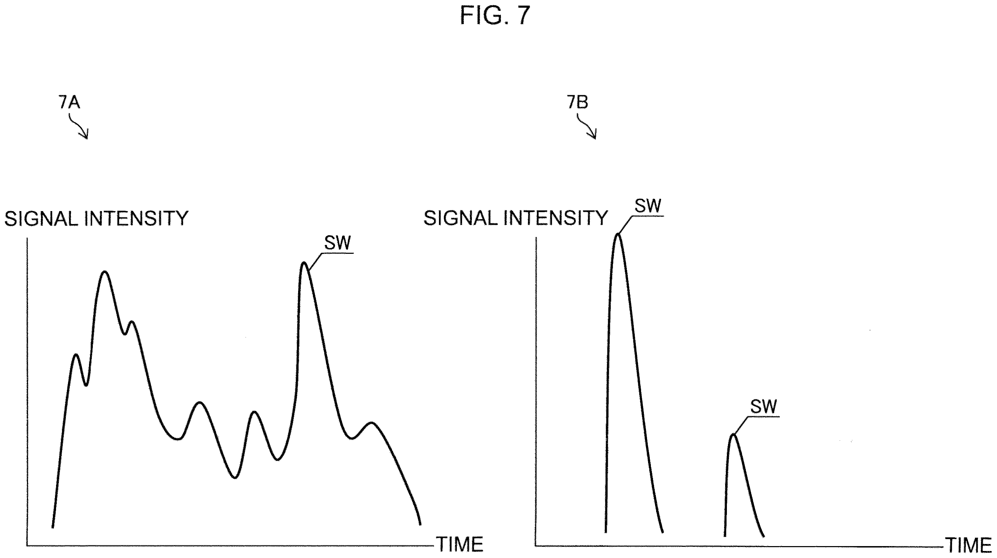

The intraocular pressure value calculating unit 44 calculates an intraocular pressure value of the subject eye E based on applanation signals continuously output from the applanation detecting optical system 24 and a detection result by the pressure sensor 34c. For example, the intraocular pressure value calculating unit 44 calculates the center of gravity or a maximum value of the peak of the applanation waveform SW based on the applanation signal output from the applanation detecting optical system 24 (see reference number 7B in Fig. 7). Then, the intraocular pressure value calculating unit 44 calculates an intraocular pressure value of the subject eye E by a publicly known method based on the signal intensity (quantity of light) of an applanation signal and a detection result (pressure of the air) by the pressure sensor 34c corresponding to the center of gravity or a maximum value of the peak. It should be noted that the method for calculating an intraocular pressure value of the subject eye E is not limited to the aforementioned method, and various kinds of methods that are publicly known can be employed.

### Operations in First Embodiment

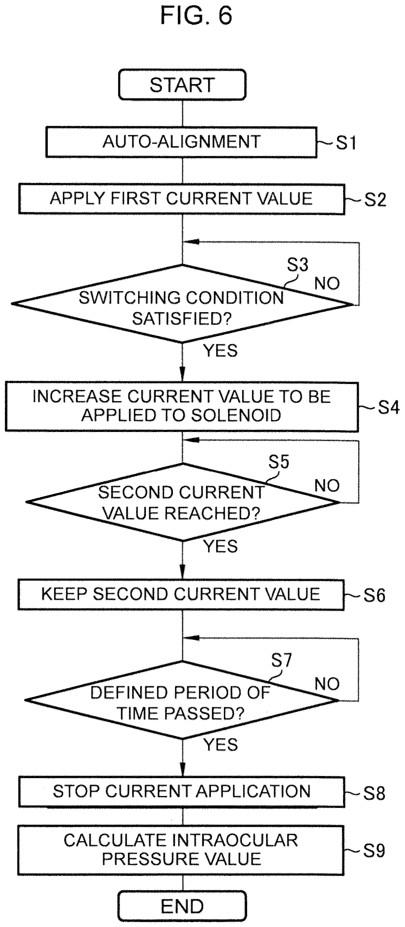

Fig. 6 is a flowchart illustrating a flow of intraocular pressure measurement processing on a subject eye E that is a method for actuating a contactless ophthalmotonometer 10 according to the first embodiment of the aforementioned configuration.

As illustrated in Fig. 6, when the tester inputs an intraocular pressure measurement start operation to an operating unit, not illustrated, after the face of the subject is set on the face supporting unit 12, the control device 16 functions as the alignment control unit 40, the measurement control unit 42, and the intraocular pressure value calculating unit 44.

At first, the alignment control unit 40 controls the anterior eye part observing optical system 21 and the XY alignment indicator projecting optical system 22 so as to perform XY alignment detection and controls the Z alignment indicator projecting optical system 25 and the Z alignment detecting optical system 26 so as to perform Z alignment detection. Then, based on the XYZ alignment detection result, the alignment control unit 40 drives the moving mechanism 13 so as to perform an auto-alignment in the XYZ directions of the measurement head 14 relative to the subject eye E (step S1).

The measurement control unit 42 actuates the spraying mechanism 34 after completion of the auto-alignment to perform spray processing. Here, the measurement control unit 42, upon initial operation of the spray processing, starts first application processing for applying current of the first current value DAC1 to the solenoid 34g (step S2). Also, the measurement control unit 42 monitors the pressure detected by the pressure sensor 34c and keeps the first application processing until the pressure achieves a pressure threshold, that is, until a switching condition is satisfied (NO in step S3). Since the lower current of the first current value DAC1 is being applied to the solenoid 34g in this stage, noise due to the spraying mechanism 34 can be reduced.

Then, when the pressure detected by the pressure sensor 34c achieves the pressure threshold while the first application processing is being performed, that is, when the switching condition is satisfied (YES in step S3), the measurement control unit 42 performs switching from the first application processing to the second application processing by increasing the current value to be applied to the solenoid 34g to the second current value DAC2 (steps S4, S5). Thus, as illustrated in Fig. 5 described above, the slope of the internal pressure waveform PW1 before and after the switching timing TA can be kept substantially constant, and the pressure of the air inside the chamber 34a can be increased sufficiently.

When the rotation angle of the solenoid 34g detected by the angle detection sensor 34i achieves an angle threshold while the first application processing is being performed, the measurement control unit 42 may determine that the switching condition is satisfied and perform switching from the first application processing to the second application processing.

The measurement control unit 42 continues the second application processing until a defined period of time passes from start of the first application processing and keeps the current value to be applied to the solenoid 34g at the second current value DAC2 (step S6 and NO in step S7). Then, when the defined period of time passes (YES in step S7), the measurement control unit 42 stops the current application to the solenoid 34g to stop the second application processing (step S8).

Also, while the spray processing is being performed, the measurement control unit 42 continuously causes processing of projecting XY-alignment indicating light to the cornea Ec by the XY alignment indicator projecting optical system 22 and signal output processing including receiving XY indicator reflected light and outputting an applanation signal by the applanation detecting optical system 24 to be performed.

When the spray processing completes, the intraocular pressure value calculating unit 44 calculates the center of gravity or a maximum value of the peak of the applanation waveform SW based on the applanation signal output from the applanation detecting optical system 24. Then, the intraocular pressure value calculating unit 44 calculates an intraocular pressure value of the subject eye E based on the signal intensity (quantity of light) of an applanation signal and a detection result (pressure of the air) by the pressure sensor 34c corresponding to the center of gravity or a maximum value of the peak (step S9).

Fig. 7 is a graph illustrating an applanation waveform SW based on an applanation signal output continuously from the applanation detecting optical system 24. The reference numeral 7A in Fig. 7 designates an example of an applanation waveform SW acquired when current having the current waveform CW2 in the comparative example illustrated in Fig. 5 described above is applied to the solenoid 34g. Also, the reference numeral 7B in Fig. 7 designates an example of the applanation waveform SW acquired when current having the current waveform CW1 in this embodiment illustrated in Fig. 5 described above is applied to the solenoid 34g.

In a case where the current value of the current to be applied to solenoid 34g is fixed to the first current value DAC1 only for the purpose of the reduction of noise due to the spraying mechanism 34, the pressure of the air within the chamber 34a does not increase sufficiently like the internal pressure waveform PW2 illustrated in Fig. 5 described above. As a result, as designated by reference numeral 7A in Fig. 7, the applanation waveform SW is not a normal waveform (see reference numeral 7B in Fig. 7), and detection of a peak from the applanation waveform SW is difficult. Thus, the precision of the measurement of an intraocular pressure value of the subject eye E decreases.

On the other hand, according to this embodiment, as designated by reference numeral 7B in Fig. 7, the current value of the current to be applied to the solenoid 34g in the middle of the spray processing is increased from the first current value DAC1 to the second current value DAC2 so that the pressure of the air within the chamber 34a can be sufficiently increased. As a result, since the applanation waveform SW is a normal waveform as designated by reference numeral 7B in Fig. 7, the intraocular pressure value calculating unit 44 can detect a peak of the applanation waveform SW with high precision. Thus, the precision of the measurement of an intraocular pressure value of the subject eye E is prevented from decreasing.

In this way, the contactless ophthalmotonometer 10 of the first embodiment seeks to reduce the noise due to the spraying mechanism 34 by applying the current of the first current value DAC1 to the solenoid 34g upon initial operation of spray processing and increases the current value to be applied to the solenoid 34g to the second current value DAC2 in the middle of the spray processing so that a decrease in precision of the measurement of an intraocular pressure value of the subject eye E can be prevented. As a result, the contactless ophthalmotonometer 10 of the first embodiment enables both of noise reduction during measurement of an intraocular pressure and precision of the measurement.

### Second Embodiment

In a case where a defined period of time that is determined in advance has passed from start of the first application processing (period of time corresponding to a pulse width W), the contactless ophthalmotonometer 10 according to the first embodiment stops the second application processing. On the other hand, the contactless ophthalmotonometer 10 of the second embodiment stops the second application processing in stop timing different from that of the first embodiment.

It should be noted that, since the contactless ophthalmotonometer 10 in the second embodiment has basically the same configuration as that of the contactless ophthalmotonometer 10 in the first embodiment, like references are given to those having the same function or configuration as that of the first embodiment, and repetitive description is omitted.

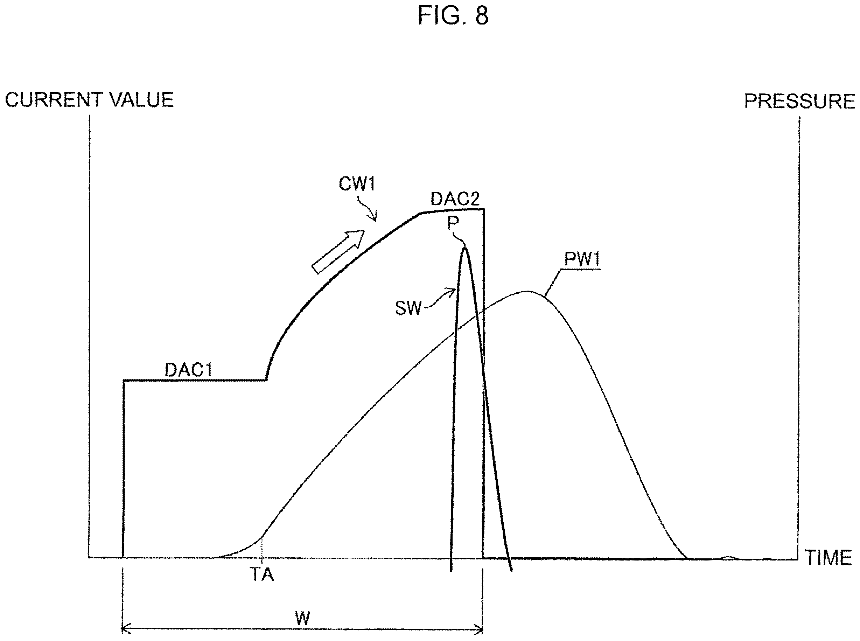

Fig. 8 is an explanatory diagram for explaining a stop timing of second application processing by the contactless ophthalmotonometer 10 according to a second embodiment. As illustrated in Fig. 8, the measurement control unit 42 in the contactless ophthalmotonometer 10 of the second embodiment continuously calculates a slope of an applanation waveform SW, for example, based on an applanation signal continuously output from the applanation detecting optical system 24 while the second application processing is being performed to detect a peak P of the applanation waveform SW. Then, when the peak P of the applanation waveform SW is detected, the measurement control unit 42 of the second embodiment stops current application to the solenoid 34g to stop the second application processing.

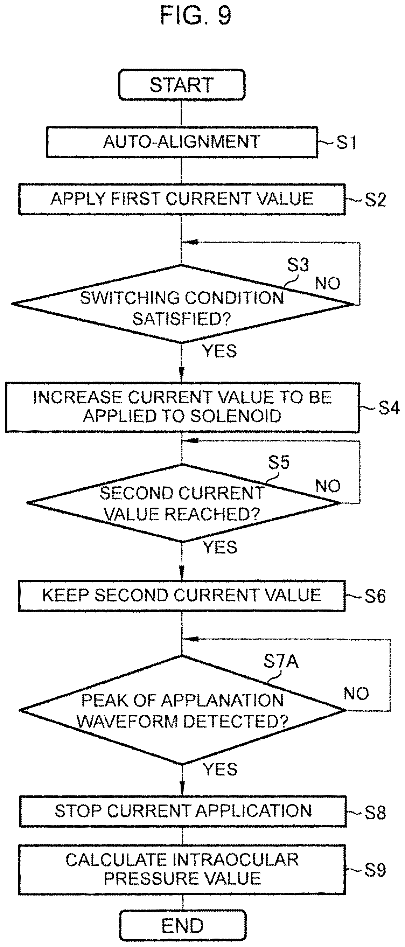

Fig. 9 is a flowchart illustrating a flow of intraocular pressure measurement processing on a subject eye E by a contactless ophthalmotonometer 10 according to the second embodiment. It should be noted that, since the processing from step S1 to step S6 is the same as the processing from step S1 to step S6 of the first embodiment illustrated in Fig. 6 described above, specific description is omitted here.

The measurement control unit 42 continues the second application processing until a peak P of the applanation waveform SW is detected based on the applanation signal continuously output from the applanation detecting optical system 24 while the second application processing is being performed and keeps the current value of the current to be applied to the solenoid 34g at the second current value DAC2 (step S6 and NO in step S7A). Then, when a peak P of the applanation waveform SW is detected (YES in step S7A), the measurement control unit 42 stops the current application to the solenoid 34g to stop the second application processing (step S8). Since the processing in subsequent steps illustrated in Fig. 6 described above is the same as the processing of the first embodiment, specific description is omitted here.

In this way, the contactless ophthalmotonometer 10 of the second embodiment also applies the current of the first current value DAC1 to the solenoid 34g upon initial operation of spray processing to increase the current value of the current to be applied to the solenoid 34g to the second current value DAC2 in the middle of the spray processing like the first embodiment so that the same effect as that of the first embodiment can be acquired. Also, by stopping the second application processing after detection of a peak P of the applanation waveform SW, so-called soft puff that reduces the amount of air to be sprayed to the subject eye E can be achieved so that unpleasantness of the subject can be reduced.

### Third Embodiment

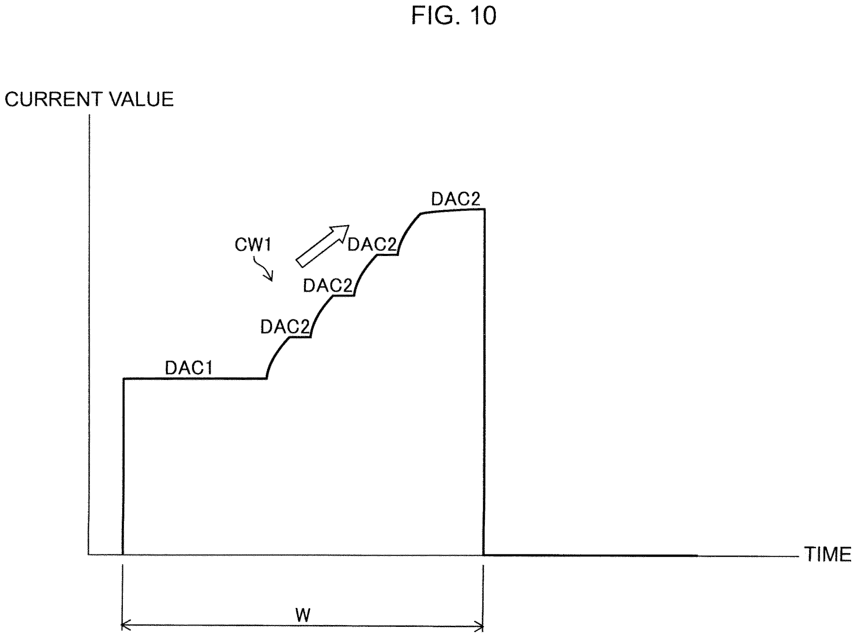

Fig. 10 is an explanatory diagram for explaining the second application processing to be performed by a contactless ophthalmotonometer 10 according to a third embodiment. In the contactless ophthalmotonometer 10 of each of the aforementioned embodiments, one set value is applied for the second current value DAC2 during the second application processing. Thus, the measurement control unit 42 of each of the aforementioned embodiments increases the current value of the current to be applied to the solenoid 34g to the one second current value DAC2 to keep it at the second current value DAC2 when the switching condition to the second application processing is satisfied while the first application processing is being performed. On the other hand, the contactless ophthalmotonometer 10 of the third embodiment performs second application processing different from that of each of the aforementioned embodiments.

Since the contactless ophthalmotonometer 10 in the third embodiment has basically the same configuration as that of the contactless ophthalmotonometer 10 in each of the aforementioned embodiments except that the method for the second application processing is different, like references are given to those having the same function or configuration as that of each of the aforementioned embodiments, and repetitive description is omitted.

As illustrated in Fig. 10, in the contactless ophthalmotonometer 10 of the third embodiment, second current values DAC2 are set. Then, the measurement control unit 42 in the contactless ophthalmotonometer 10 of the third embodiment increases the second current value DAC2 of the current to be applied to the solenoid 34g in a stepwise manner through steps when the switching condition to the second application processing is satisfied while the first application processing is being performed.

More specifically, a pressure threshold as described above is set in advance for each of the second current values DAC2. Here, as the second current value DAC2 increases in a stepwise manner, the pressure threshold is set to be higher in a stepwise manner. Then, the measurement control unit 42 applies current of the second current value DAC2 corresponding to the pressure threshold to the solenoid 34g every time the pressure detected by the pressure sensor 34c while the second application processing is being performed achieves the pressure threshold. Thus, the measurement control unit 42 can increase the second current value DAC2 in a stepwise manner in accordance with an increase in the pressure detected by the pressure sensor 34c while the second application processing is being performed.

Also, an angle threshold as described above may be set in advance for each of the second current values DAC2. Here, as the second current value DAC2 increases in a stepwise manner, the angle threshold is set to be higher in a stepwise manner. Then, the measurement control unit 42 applies current of the second current value DAC2 corresponding to the angle threshold to the solenoid 34g every time the rotation angle of the solenoid 34g detected by the angle detection sensor 34i while the second application processing is being performed achieves the angle threshold. Thus, the measurement control unit 42 can increase the second current value DAC2 in a stepwise manner in accordance with an increase in the rotation angle of the solenoid 34g detected by the angle detection sensor 34i while the second application processing is being performed.

The measurement control unit 42 can increase the second current value DAC2 of the current to be applied to the solenoid 34g in a stepwise manner every time the pressure detected by the pressure sensor 34c or the rotation angle detected by the pressure sensor 34c increases by a predetermined amount while the second application processing is being performed.

In this way, the contactless ophthalmotonometer 10 of the third embodiment also applies the current of the first current value DAC1 to the solenoid 34g upon initial operation of spray processing to increase the second current value DAC2 of the current to be applied to the solenoid 34g in a stepwise manner in the middle of the spray processing so that the same effect as that of each of the aforementioned embodiments can be acquired.

### Others

While the piston 34f is driven by the solenoid 34g in each of the aforementioned embodiments, a rotary actuator other than the solenoid 34g may be used to drive the piston 34f, or even an actuator other than a rotary actuator may be used when the piston 34f can be driven.

While each of the aforementioned embodiments is described with reference to the contactless ophthalmotonometer 10 as an example, the aspects of the technology described herein is also applicable to a multifunction apparatus which can measure an intraocular pressure value of a subject eye E and an ocular characteristic other than the intraocular pressure value of the subject eye E.

In any of the embodiments of the disclosure provided herein, the contactless ophthalmotonometer 10 may include one or more computer hardware processors and one or more articles of manufacture that include non-transitory computer-readable storage media (e.g., memory and one or more non-volatile storage devices). The processor(s) may control writing data to and reading data from the memory and the non-volatile storage device(s) in any suitable manner. To perform any of the functionality described herein, the processor(s) may execute one or more processor-executable instructions stored in one or more non-transitory computer-readable storage media (e.g., the memory), which may serve as non-transitory computer-readable storage media storing processor-executable instructions for execution by the processor(s).

The terms "program" or "software" are used herein in a generic sense to refer to any type of computer code or set of processor-executable instructions that can be employed to program a computer or other processor (physical or virtual) to implement various aspects of embodiments as discussed above. Additionally, according to one aspect, one or more computer programs that when executed perform methods of the disclosure provided herein need not reside on a single computer or processor, but may be distributed in a modular fashion among different computers or processors to implement various aspects of the disclosure provided herein.

Processor-executable instructions may be in many forms, such as program modules, executed by one or more computers or other devices. Generally, program modules include routines, programs, objects, components, data structures, etc. that perform particular tasks or implement particular abstract data types. Typically, the functionality of the program modules may be combined or distributed.

Also, data structures may be stored in one or more non-transitory computer-readable storage media in any suitable form. For simplicity of illustration, data structures may be shown to have fields that are related through location in the data structure. Such relationships may likewise be achieved by assigning storage for the fields with locations in a non-transitory computer-readable medium that convey relationship between the fields. However, any suitable mechanism may be used to establish relationships among information in fields of a data structure, including through the use of pointers, tags or other mechanisms that establish relationships among data elements.

Various inventive concepts may be embodied as one or more processes, of which examples have been provided. The acts performed as part of each process may be ordered in any suitable way. Thus, embodiments may be constructed in which acts are performed in an order different than illustrated, which may include performing some acts simultaneously, even though shown as sequential acts in illustrative embodiments.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, for example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

Use of ordinal terms such as "first," "second," "third," etc., in the claims to modify a claim element does not by itself connote any priority, precedence, or order of one claim element over another or the temporal order in which acts of a method are performed. Such terms are used merely as labels to distinguish one claim element having a certain name from another element having a same name (but for use of the ordinal term). The phraseology and terminology used herein is for the purpose of description and should not be regarded as limiting. The use of "including," "comprising," "having," "containing," "involving," and variations thereof, is meant to encompass the items listed thereafter and additional items.

Having described several embodiments of the techniques described herein in detail, various modifications, and improvements will readily occur to those skilled in the art. Such modifications and improvements are intended to be within the spirit and scope of the disclosure. Accordingly, the foregoing description is by way of example only, and is not intended as limiting. The techniques are limited only as defined by the following claims and the equivalents thereto.

### Reference Signs List

- 10: contactless ophthalmotonometer
- 11: base
- 12: face supporting unit
- 12a: jaw receiver
- 12b: forehead protector
- 12c: support rod
- 13: moving mechanism
- 14: measurement head
- 15: monitor
- 16: control device
- 21: anterior eye part observing optical system
- 21a: anterior eye part illumination light source
- 21b: nozzle
- 21c: anterior eye part window glass
- 21d: chamber window glass
- 21e: half-mirror
- 21f: objective lens
- 21g: half-mirror
- 21i: image-capturing element
- 22: XY alignment indicator projecting optical system
- 22a: XY alignment light source
- 22b: condensing lens
- 22c: aperture diaphragm
- 22d: pinhole plate
- 22e: dichroic mirror
- 22f: collimator lens
- 23: fixation target projecting optical system
- 23a: fixation target light source
- 23b: pinhole plate
- 24: applanation detecting optical system
- 24a: lens
- 24b: pinhole plate
- 24c: received light sensor
- 25 Z: alignment indicator projecting optical system
- 25a: Z alignment light source
- 25b: condensing lens
- 25c: aperture diaphragm
- 25d: pinhole plate
- 25e: collimator lens
- 26: Z alignment detecting optical system
- 26a: image-forming lens
- 26b: cylindrical lens
- 26c: received light sensor
- 34: spraying mechanism
- 34a: chamber
- 34b: glass plate
- 34c: pressure sensor
- 34d: cylinder
- 34e: communicating tube
- 34f: piston
- 34g: solenoid
- 34h: crank mechanism
- 34i: angle detection sensor
- 40: alignment control unit
- 42: measurement control unit
- 44: intraocular pressure value calculating unit
- CW1: current waveform
- CW2: current waveform
- DAC1: first current value
- DAC2: second current value
- E: subject eye
- Ec: cornea
- Ep: cornea vertex
- O1: optical axis
- 02: optical axis
- 03: optical axis
- P: peak
- PW1: internal pressure waveform
- PW2: internal pressure waveform
- SW: applanation waveform
- TA: switching timing
- W: pulse width

## Claims

1. A contactless ophthalmotonometer (10), comprising:
a cylinder (34d);
a piston (34f) movably provided inside the cylinder (34d);
an actuator (34g) configured to move the piston (34f) inside the cylinder (34d) with current applied to the actuator to compress air inside the cylinder (34d) with the piston (34f);
a nozzle (21b) in communication with the inside of the cylinder (34d), the nozzle configured to spray the air compressed with the piston (34f) to a subject eye; and
a current application control unit (42) configured to apply the current to the actuator (34g),
wherein the current application control unit (42) performs first application processing for applying the current of a first current value to the actuator (34g) and, when a predetermined switching condition is satisfied while the first application processing is being performed, the first application processing is switched to second application processing for applying the current of a second current value higher than the first current value to the actuator (34g).

2. The contactless ophthalmotonometer (10) according to claim 1,
wherein the nozzle (21b) is in communication with the inside of the cylinder (34d) through a chamber in communication with the inside of the cylinder (34d),
the contactless ophthalmotonometer (10) further comprises a pressure sensor (34c) configured to detect pressure inside the chamber, and
the current application control unit (42) determines whether the switching condition is satisfied or not based on whether the pressure detected by the pressure sensor (34c) achieves a predetermined pressure threshold.

3. The contactless ophthalmotonometer (10) according to claim 1, further comprising:
a detection sensor (34i) configured to detect a displaced amount of the actuator (34g),
wherein the current application control unit (42) determines whether the switching condition is satisfied or not based on whether the displaced amount detected by the detection sensor (34i) achieves a predetermined displaced amount threshold.

4. The contactless ophthalmotonometer (10) according to any one of claims 1 to 3,
wherein the current application control unit (42) increases the second current value in a stepwise manner while the second application processing is being performed.

5. The contactless ophthalmotonometer (10) according to claim 4,
wherein the nozzle (21b) is in communication with the inside of the cylinder (34d) through a chamber in communication with the inside of the cylinder,
the contactless ophthalmotonometer (10) further comprises a pressure sensor (34c) configured to detect pressure inside the chamber, and
the current application control unit (42) increases the second current value in a stepwise manner in accordance with an increase in the pressure detected by the pressure sensor (34c) while the second application processing is being performed.

6. The contactless ophthalmotonometer (10) according to claim 4, further comprising:
a detection sensor (34i) configured to detect a displaced amount of the actuator (34g),
wherein the current application control unit (42) increases the second current value in a stepwise manner in accordance with an increase in the displaced amount detected by the detection sensor (34i) while the second application processing is being performed.

7. The contactless ophthalmotonometer (10) according to any one of claims 1 to 6,
wherein the current application control unit (42) stops the second application processing when a defined period of time determined in advance passes from start of the first application processing.

8. The contactless ophthalmotonometer (10) according to any one of claims 1 to 7, further comprising:
a projecting optical system (22) configured to project a luminous flux to the subject eye while the air is being sprayed from the nozzle (21b) to the subject eye; and
a receiving optical system (24) configured to receive reflected light of the luminous flux reflected at the subject eye and outputs an applanation signal while the air is being sprayed from the nozzle (21b) to the subject eye,
wherein the current application control unit (42) stops the second application processing when a peak of the applanation signal output from the receiving optical system (24) is detected while the second application processing is being performed.

9. The contactless ophthalmotonometer (10) according to any one of claims 1 to 8,
wherein the actuator (34g) is a rotary actuator configured to cause the piston (34f) to move inside the cylinder (34d) through a crank mechanism.

10. A method for actuating a contactless ophthalmotonometer (10), the contactless ophthalmotonometer (10) including:
a cylinder (34d);
a piston (34f) movably provided inside the cylinder (34d);
an actuator (34g) configured to move the piston (34f) inside the cylinder (34d) with current applied to the actuator to compress air inside the cylinder (34d) with the piston (34f);
a nozzle (21b) in communication with the inside of the cylinder (34d), the nozzle configured to spray the air compressed with the piston (34f) to a subject eye,
the method including:
performing first application processing for applying the current of a first current value to the actuator (34g) when the current is to be applied to the actuator (34g); and
switching the first application processing to second application processing for applying the current of a second current value higher than the first current value to the actuator (34g) when a predetermined switching condition is satisfied while the first application processing is being performed.
